# FASCICULE DE BREVET EUROPEEN

(11) **EP 1 583 478 B1**
(45) Date de publication et mention de la délivrance du brevet: **23.12.2009**
(21) Numéro de dépôt: 04702350.2
(22) Date de dépôt: 15.01.2004
(51) Int. Cl.: A61B 17/80, A61B 17/60

(54) **DISPOSITIF DE BLOCAGE D'UNE BROCHE D'OSTEOSYNTHESE DANS UNE PIECE OSSEUSE**
VORRICHTUNG ZUR IMMOBILISIERUNG EINES OSTEOSYNTHESESTIFTS IN EINEM KNOCHENTEIL
DEVICE FOR IMMOBILISING AN OSTEOSYNTHESIS PIN IN A BONE PART

(30) Priorité: 15.01.2003 FR 0300393
(43) Date de publication de la demande: 12.10.2005
(73) Titulaire: Dongar, Christian, 95270 Viarmes (FR); Worcel, Alexandre, Parc de Mongarny 95680 Montlignon (FR)
(72) Inventeur: Dongar, Christian, 95270 Viarmes (FR); Worcel, Alexandre, Parc de Mongarny 95680 Montlignon (FR)
(74) Mandataire: Puiroux, Guy
(86) Numéro de dépôt international: PCT/FR2004/000070
(87) Numéro de publication internationale: WO 2004/069067

(56) Documents cités:
- EP-A- 0 146 872
- WO-A-01/12081
- DE-A- 4 428 518
- US-A- 5 304 179
- US-A- 5 393 161
- US-B1- 6 280 445

## Description

La présente invention concerne un dispositif de blocage sur un élément fixe, notamment constitué d'une plaque d'ostéosynthèse, d'une broche filetée de maintien vissée dans une pièce osseuse.

On connaît divers dispositifs de l'état antérieur de la technique qui sont en mesure d'assurer une telle fixation. On a ainsi proposé d'utiliser des vis de type classique dont la tête est fraisée à 45°, ce qui permet de la noyer à l'intérieur d'un évidement de même forme prévue dans la plaque, si bien qu'il n'existe à l'extérieur de celle-ci aucune partie agressive ou contondante. On a proposé dans une variante de mise en oeuvre d'obturer la partie supérieure de l'évidement contenant la tête de la vis à l'aide d'un écrou en forme de pastille qui vient se visser dans un filetage correspondant prévu à l'entrée dudit évidement. Si un tel dispositif permet d'éviter toute saillie ou toute discontinuité de surface avec celle de la plaque, il présente cependant l'inconvénient, dans le cas où le filetage de cet écrou serait endommagé, d'empêcher tout démontage de la vis de fixation.

Un tel dispositif présente par ailleurs également l'inconvénient, lorsque l'axe du trou fileté réalisé dans la partie osseuse n'est pas rigoureusement confondu avec celui de ladite cavité tronconique, de créer des contraintes au niveau de la partie osseuse, contraintes susceptibles au cours du temps de porter atteinte à l'intégrité de la partie osseuse et en conséquence de nuire à la bonne fixation de la plaque.

Afin d'éviter un tel inconvénient, on a proposé de doter la plaque d'un élément intermédiaire constituant une sorte de rotule sphérique à l'intérieur de laquelle est prévu le logement tronconique destiné à recevoir la tête de la vis de fixation. On comprend dans ces conditions que la rotule sphérique permet de compenser les défauts d'alignement de ladite cavité avec l'axe du trou récepteur de la vis. Si un tel dispositif permet ainsi d'éviter les contraintes mécaniques précédemment mentionnées se produisant au niveau de la plaque et de la partie osseuse, il présente néanmoins l'inconvénient, dans la mesure où il créée un nouveau degré de liberté entre la plaque et la vis, d'éviter "l'effet de structure bloc" qui se produit lorsque plusieurs vis de fixation sont solidaires d'une même plaque. En effet dans un tel cas, on a constaté que, même si les vis de fixation sont peut serrées sur la partie osseuse, le fait que ces vis soient solidaires de la plaque et s'enfoncent dans la partie osseuse suivant des axes différents, constitue une sorte de système hyperstatique assurant une solidarisation de la plaque. Or une telle disposition est particulièrement intéressante dans la mesure où, le plus souvent, les pièces osseuses dans lesquelles sont positionnées les vis relèvent de pathologies portant atteinte à leur qualité mécanique, si bien que non seulement la mise en place de ces vis ne renforce pas cette qualité mécanique mais au contraire a plutôt tendance à l'amoindrir, voire à la supprimer totalement lorsque l'on exerce un effort de serrage trop important.

On remarquera par ailleurs que, dans le cas de fractures ouvertes, de tels dispositifs de maintien sont totalement inopérants, puisqu'ils doivent alors être éloignés de la surface de la peau du patient, si bien qu'il n'est pas possible alors de faire appel à des plaques d'ostéosynthèse.

Enfin, les différents dispositifs de maintien de l'état antérieur de la technique qui font appel à des vis présentent la contrainte d'obliger le praticien à disposer d'un assortiment complet de celles-ci aussi bien en ce qui concerne leur longueur que leur diamètre. C'est pourquoi les centres d'intervention hospitaliers sont contraints de disposer, contrôler et maintenir à disposition un stock important de vis ce qui représente un coût important d'une part en ce qui concerne la matière première elle-même et d'autre part également en ce qui concerne la logistique.

La présente invention a pour but de proposer un dispositif de blocage permettant à un praticien d'une part d'ajuster la longueur des vis utilisées en fonction de l'application, ce qui évite le problème du stockage des vis, et d'autre part permet d'assurer le blocage des pièces osseuses dans le cas de fractures ouvertes.

La présente invention a ainsi pour objet un dispositif de blocage par rapport à un élément fixe d'une broche de maintien filetée vissée dans une pièce osseuse suivant un axe, caractérisé en ce qu'il comporte une douille de blocage solidarisable par vissage de l'élément fixe, qui est pourvue d'un alésage axial qui reçoit la broche, l'axe de l'alésage de la douille de blocage est décalé angulairement et/ou latéralement par-rapport à l'axe du filetage.

Afin de favoriser le démontage du dispositif une pièce intermédiaire apte à être fixée sur l'élément fixe pourra être disposée entre ce dernier et la douille de blocage.

Dans un mode de mise en oeuvre de l'invention la pièce intermédiaire pourra être constituée d'une douille pourvue d'un filetage externe par lequel elle est vissée dans l'élément fixe, l'axe du filetage externe de cette douille intermédiaire étant confondu avec l'axe de la broche lorsque celle-ci est en place sur la pièce osseuse. L'élément fixe quant à lui pourra être constitué d'une plaque qui sera appliquée contre la pièce osseuse et sera destinée à être solidarisée de celle-ci.

Suivant l'invention la partie filetée de la douille de blocage pourra se prolonger vers l'extérieur par un élément de préhension d'entraînement en rotation de celle-ci, et une zone de moindre résistance à niveau de rupture en cisaillement contrôlée sera prévue entre ladite partie filetée et l'élément de préhension. La douille intermédiaire pourra comporter à sa partie supérieure un bossage circulaire formant butée, destiné à venir en appui sur la plaque, notamment dans un chambrage de celle-ci. Ce bossage circulaire pourra comporter une série d'orifices destinés à assurer sa préhension et son entraînement en rotation afin de bloquer/débloquer la douille intermédiaire sur la plaque.

Dans un autre mode de mise en oeuvre de l'invention l'élément fixe sera constitué d'un support récepteur de pièces intermédiaires qui seront montées mobiles d'une part suivant le profil de celui-ci, et d'autre part en rotation, des moyens de fixation permettant d'assurer l'immobilisation des pièces intermédiaires par rapport à l'élément fixe. Cet élément fixe pourra notamment être constitué d'un rail dont le profil interne sera de forme hémisphérique et dont la base sera percée d'une lumière longitudinale apte à être traversée par les broches filetées. Les pièces intermédiaires seront constituées de sphères aptes à être positionnées en un point quelconque du rail, et le dispositif il comportera un capot supérieur de profil interne hémisphérique -dont le sommet sera percé d'une fente longitudinale, apte à être traversée par les brochures filetées, et des moyens de serrage aptes à appliquer les pièces intermédiaires sphériques contre le rail pour les immobiliser à la fois en translation et en rotation par rapport à celui-ci

On décrira ci-après, à titre d'exemple non limitatif, une forme d'exécution de la présente invention, en référence au dessin annexé sur lequel :
La figure 1 est une vue en coupe longitudinale d'un dispositif de blocage suivant l'invention qui est mis en place sur une pièce osseuse.
La figure 2 est une vue en coupe transversale du dispositif représenté sur la figure 1, suivant la ligne II-II de celle-ci.
La figure 3 est une vue schématique d'un second exemple d'application du dispositif de blocage suivant l'invention.
La figure 4 est une variante de réalisation du second exemple d'application de la présente invention représenté sur la figure 3.
La figure 5 est une vue en coupe partielle de la variante de mise en oeuvre représentée sur la figure 4 suivant la ligne V-V de celle-ci.
La figure 6 est une vue en coupe à échelle agrandie d'un détail de mise en oeuvre représenté sur la figure 4.

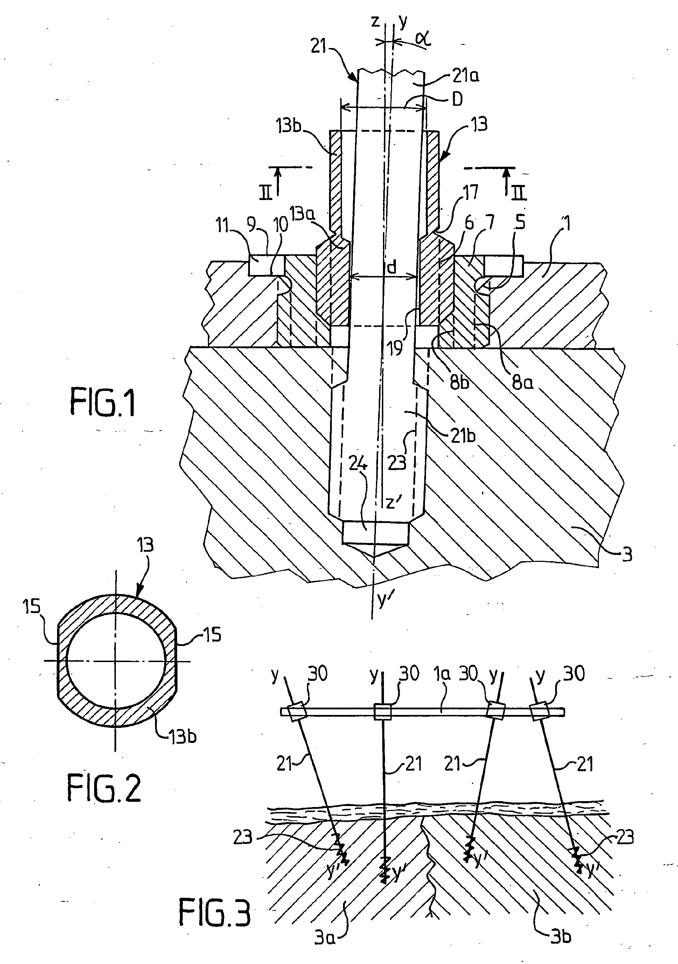

Le dispositif de blocage représenté sur la figure 1 a pour but d'assurer la solidarisation d'une plaque d'ostéosynthèse 1 sur une pièce osseuse 3. A cet effet, la plaque 1 est percée d'un orifice fileté 5 dans lequel vient se visser une douille intermédiaire 7 pourvue à sa partie supérieure d'une collerette 9 comportant des encoches 11 destinées à assurer sa préhension au cours de son vissage dans la plaque 1. La collerette 9 prend place dans un chambrage circulaire 10 prévu à la partie supérieure de la plaque 1.

La douille intermédiaire 7 est elle-même percée d'un orifice central fileté 8b dont l'axe zz' est incliné d'un angle α par rapport à l'axe de révolution yy' de la douille intermédiaire 7 et de son filetage externe 8a.

Une douille de blocage 13 est vissée par un filetage 6 réalisé à sa partie inférieure filetée 13a dans la douille intermédiaire 7. La partie supérieure 13b de cette douille de blocage 13 comporte, sur deux de ses flans opposés, des méplats 15 destinés à faciliter la préhension de cette partie. Les parties supérieure 13b et inférieure 13a de cette douille 13 sont réunies par une zone de moindre résistance 17 constituée ici par une saignée circulaire.

L'orifice interne 19 de la partie inférieure 13a de la douille 13 de diamètre d reçoit à coulissement une broche 21, pourvue à son extrémité inférieure d'un filetage 23 destiné à être vissé dans la pièce osseuse 3. L'alésage interne de la partie supérieure 13b est d'un diamètre D supérieur à celui de la broche 21. Dans ces conditions, le fonctionnement du dispositif de blocage suivant l'invention s'effectue ainsi que décrit ci-après.

Le praticien, après avoir repéré la position de la plaque 1 qu'il souhaite fixer sur la pièces osseuse 3 et avoir vissé dans celle-ci un guide de perçage, non représenté sur le dessin, effectue un perçage 24 en guidant son foret dans ce guide dont l'axe longitudinal yy' est perpendiculaire à la plaque.

Après avoir remplacé dans la plaque 1 le guide de perçage par la douille intermédiaire 7, le praticien visse la broche filetée 21 dans la pièce osseuse à la profondeur souhaitée. Il introduit ensuite la douille de blocage 13 sur la tige filetée 21 et procède au vissage de celle-ci dans la douille intermédiaire 7, en s'aidant pour ce faire des deux méplats de préhension 15 prévus sur la paroi externe de la partie supérieure 13b de celle-ci.

On comprend, qu'en raison du décalage angulaire α existant entre les deux axes respectifs yy' et zz', on réalise au cours de ce vissage, un coincement progressif de la douille 13 dans la douille intermédiaire 7 aboutissant à un véritable blocage créant un effet de sertissage, tel que lorsque celui-ci s'est produit, il n'est plus possible alors de dévisser la douille de blocage 13 de la douille intermédiaire 7. Le blocage dont il s'agit est du type de celui se produisant parfois lorsqu'un élément fileté de pas particulièrement fin est mis en place de travers dans un écrou, aboutissant à une liaison quasiment indémontable sauf rupture de l'élément fileté. Dans le cas présent, la zone de moindre résistance, constituée ici par l'étranglement 17, sera déterminée de façon telle que lorsqu'un couple souhaité C est atteint, on provoque la rupture de la partie supérieure 13b de la douille 13.

Il restera ensuite au praticien à procéder à la mise à longueur de la broche 21, en réalisant un cisaillage de celle-ci au plus près de la partie supérieure 13b de la pièce 13.

On obtient ainsi un blocage irréversible de la broche 21 par rapport à la plaque 1.

Le démontage nécessaire à des interventions ultérieures est assuré par le dévissage de la douille intermédiaire 7.

On notera que, suivant l'invention, à l'inverse des dispositifs de fixation de plaque de l'état antérieur de la technique, cette fixation de la plaque sur la pièce osseuse 3 s'effectue en exerçant des contraintes minimales sur celle-ci, ce qui permet de respecter les pièces osseuses qui, la plupart du temps, pour diverses raisons, ont des caractéristiques mécaniques amoindries.

Suivant la présente invention, également à l'inverse des dispositifs de l'état antérieur de la technique, le blocage irréversible crée a pour effet de réunir en un seul bloc la plaque 1 et la broche filetée 21 ce qui est particulièrement intéressant dans la mesure où, même si la partie osseuse voit sa résistance mécanique s'amoindrir au cours du temps, les différentes vis de fixation assurent un maintien de la pièces osseuse. Cette structure solidaire de la pièce osseuse est d'autant plus efficace que la plaque 1 est maintenue sur celle-ci par des broches 21 dont les axes ne sont pas parallèles.

La présente invention est également intéressante pour réduire des fractures ouvertes, c'est-à-dire pour assurer le maintien de plusieurs parties osseuses sans qu'il soit possible d'appliquer à la surface de la peau une plaque de maintien.

On a représenté de façon schématique sur la figure 3 le principe de maintien de deux éléments de pièces osseuses 3a et 3b au moyen d'un dispositif suivant l'invention. Celui-ci est essentiellement constitué d'un élément de maintien rigide 1a, sur lequel sont montés à coulissement des éléments de blocage 30 aptes à assurer la fixation de broches filetées 21 qui sont vissées dans les éléments de pièces osseuses 3a et 3b.

Préférentiellement, le dispositif de fixation des broches sur l'élément de maintien autorise un positionnement en rotation de ces broches de façon à permettre au praticien une grande liberté de choix en ce qui concerne la localisation de leur vissage dans les pièces osseuses 3a, 3b.

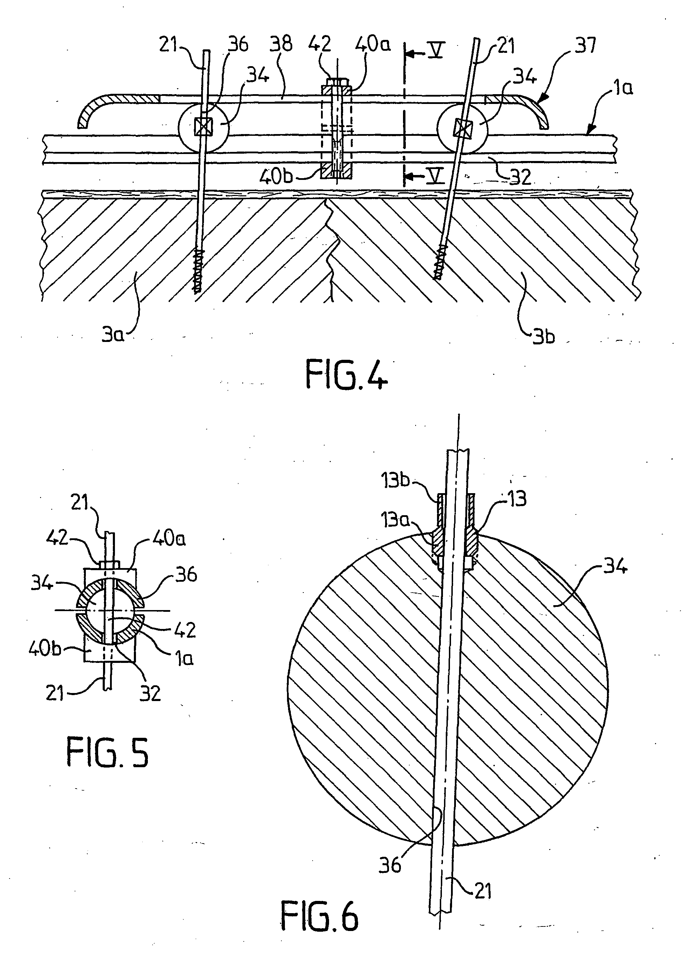

On a représenté sur les figures 4 et 5 une variante de mise en oeuvre d'un dispositif du type de celui représenté sur la figure 3.

Ce dispositif comprend ainsi un rail la constitué d'un élément métallique profilé dont la section droite interne est de forme semi-circulaire et dont le fond est creusé d'une lumière longitudinale 32, si bien qu'il constitue une sorte de goulotte. A l'intérieur de celle-ci, on dispose des sphères métalliques 34 qui sont percées d'un trou diamétral 36 permettant de les enfiler sur la partie non filetée de broches de fixation 21. Les sphères 34 sont pourvues d'un dispositif de blocage/sertissage du type de celui représenté sur les figures 1, 2 et 6, et qui permet d'assurer leur solidarisation des broches 21. Ce dispositif permet ainsi au chirurgien de positionner les broches de fixation dans les parties osseuses 3a et 3b dans un endroit de son choix puisqu'il aura d'une part la possibilité de faire coulisser les sphères 34 dans la goulotte et d'autre part la possibilité de faire pivoter l'ensemble broche/sphère suivant un angle de son choix.

Une fois les différentes broches de fixation vissées dans les parties osseuses 3a et 3b, on assurera la solidarisation irréversible des sphères 34 et des broches 21 respectives ainsi qu'exposé dans le mode de mise en oeuvre décrit précédemment, puis on assurer l'immobilisation de chacun de ces ensembles par rapport au rail de maintien 1a en recouvrant les sphères par un capot longitudinal 37 à section interne semi sphérique et pourvu également d'une lumière longitudinale 38 destinée à laisser passer les broches 21, que l'on appliquera fortement contre le rail 1a au moyen d'un étrier en deux éléments 40a, 40b qui seront appliqués fortement l'un contre l'autre par une vis de serrage 42.

## Revendications

1. Dispositif de blocage par rapport à un élément fixe (1,34) d'une broche de maintien filetée (21) vissée dans un perçage (24) d'axe (yy') d'une pièce osseuse (3,3a,3b), comportant une douille de blocage (13) pourvue d'un filetage externe (6) par lequel elle est solidarisable par vissage de l'élément fixe (1,34,7), la douille etant pourvues d'un alésage axial (19) qui est adapté à recevoir la broche (21), **caractérisé en ce que** l'axe de l'alésage (19) de la douille de blocage (13) est décalé angulairement et/ou latéralement par rapport à l'axe du filetage (6).

2. Dispositif suivant la revendication 1 **caractérisé en ce qu'**il comporte une pièce intermédiaire (7) apte à être fixée sur l'élément fixe (1), et apte à être disposée entre ce dernier et la douille de blocage (13).

3. Dispositif suivant la revendication 2 **caractérisé en ce que** la pièce intermédiaire est constituée d'une douille (7) pourvue d'un filetage externe (8a) par lequel elle est vissée dans l'élément fixe (1), l'axe (zz') du filetage externe (8a) de cette douille intermédiaire étant confondu avec l'axe du filetage (6).

4. Dispositif suivant l'une des revendications 1 à 3 **caracterisé en ce qu'**il comporte l'élément fixe qui est constitué d'une plaque (1) qui est applicable contre la pièce osseuse (3) et est destinée à être solidarisée de celle-ci.

5. Dispositif suivant l'une des revendications 3 ou 4 **caractérisé en ce que** la partie filetée (13a) de la douille de blocage (13) se prolonge vers l'extérieur par un élément de préhension d'entraînement en rotation (13b) de celle-ci, et une zone de moindre résistance (17) à niveau de rupture en cisaillement contrôlé est prévue entre ladite partie filetée (13a) et l'élément de préhension (13b).

6. Dispositif suivant la revendication 4, **caractérisé en ce que** la douille intermédiaire (7) comporte à sa partie supérieure un bossage circulaire (9) formant butée, destiné à venir en appui sur la plaque (1), notamment dans un chambrage (10) de celle-ci.

7. Dispositif suivant la revendication 6, **caractérisé en ce que** le bossage circulaire (9) comporte une série d'orifices (11) destinés à assurer sa préhension et son entraînement en rotation afin de bloquer/débloquer la douille intermédiaire (7) sur la plaque (1).

8. Dispositif suivant la revendication 2 **caractérisé en ce qu'**il comprend en outre un élément fixe constitué d'un support (1a) récepteur de pièces intermédiaires (30), qui sont montées mobiles d'une part suivant le profil de celui-ci, et d'autre part en rotation, des moyens de fixation (36) permettant d'assurer l'immobilisation des pièces intermédiaires (30) par rapport à l'élément fixe (1a).

9. Dispositif suivant la revendication 8 **caractérisé en ce que**:
- l'élément fixe est constitué d'un rail (1a) dont le profil interne est de forme hémisphérique et dont la base est percée d'une lumière longitudinale (32) apte à être traversée par les broches filetées (21),
- les pièces intermédiaires sont constituées de sphères (34) aptes à être positionnées en un point quelconque du rail (la),
- il comporte un capot supérieur (36) de profil interne hémisphérique dont le sommet est percé d'une fente longitudinale (38), apte à être traversée par les broches filetées (21),
- il comporte.des moyens de serrage (40a,40b,42) aptes à appliquer les pièces intermédiaires sphériques (34) contre le rail (1a) pour les immobiliser à la fois en translation et en rotation par rapport à celui-ci.

## Claims

1. Locking device relative to a fixed member (1, 34) of a threaded holding pin (21) threaded in a boring (24) of axis (yy') of a bone piece (3, 3a, 3b) comprising a locking socket (13) provided with an outer thread (6) through which it is integrally threaded into the fixed member (1, 34, 71), the socket being provided with an axial bore (19) which is adapted to receive the pin (21), **characterized in that** the bore axis (19) of the locking socket (13) is angularly and/or laterally shifted relative to the thread axis (6).

2. Device as claimed in claim 1, **characterized in that** it comprises an intermediate piece (7) suitable for being attached to the fixed member (1) and suitable for being arranged between the latter and the locking socket (13).

3. Device as claimed in claim 2, **characterized in that** the intermediate piece consists of a socket (7) provided with an outer thread (8a) through which it is threaded into the fixed member (1), the axis (zz') of the outer thread (8a) of this intermediate socket being merged with the axis of the thread (6).

4. Device as claimed in any of claim 1 to 3, **characterized in that** it comprises the fixed member which consists of a plate (1) which is applicable against the bone piece (3) and is intended to be integrally fitted therewith.

5. Device as claimed in any of claim 3 or 4, **characterized in that** the threaded part (13a) of the locking socket (13) extends outwards through a rotatingly drive gripping member (13b) thereof, and a lower resistance area (17) with controlled shearing break level is provided between the threaded part (13a) and the gripping member (13b).

6. Device as claimed in claim 4, **characterized in that** the intermediate socket (7) has, at its upper part, a circular stop forming boss (9) suitable for bearing to the plate (1) particularly in a recess (10) thereof.

7. Device as claimed in claim 6, **characterized in that** the circular boss (9) comprises a series of ports (11) provided to ensure its griping and its rotating drive so as to lock / release the intermediate socket (7) to the plate (1).

8. Device as claimed in claim 2, **characterized in that** it further comprises a fixed member consisted of a support (1a) receiving the intermediate pieces (30) which are movingly fitted on one hand according to the profile thereof, and on the other hand rotatingly fitted, attachment means (36) allowing to ensure the immobilization of the intermediate parts (30) relative to the fixed member (1a).

9. Device as claimed in claim 8, **characterized in that** :
the fixed member consists of a rail (1a) the inner profile of which is of hemispherical shape and the base of which is pierced of a longitudinal lumen (32) suitable for being crossed by the threaded pins (21),
the intermediate parts consist of spheres (34) suitable for being positioned in any point of the rail (1a),
it comprises a top cover (36) with a hemispherical inner profile, the top of which is pierced of a longitudinal slot (38) suitable for being crossed by the threaded pins (21),
it comprises clamp means (40a, 40b, 42) suitable for being applied to the spherical intermediate pieces (34) against the rail (1a) to lock them both in translation and in rotation relative to the latter.

## Patentansprüche

1. Vorrichtung zum Blockieren bezüglich eines feststehenden Elementes (1, 34) eines mit Gewinde versehenen Haltestiftes (21), der in ein Bohrloch (24) mit Mittellinie (yy') eines Knochenstücks (3, 3a, 3b) eingeschraubt ist, aufweisend eine Blockierbuchse (13), die mit einem Außengewinde (6) versehen ist, mittels dem sie durch Verschrauben mit dem festen Element (1, 34, 7) fest verbunden werden kann, wobei die Buchse mit einer Axialbohrung (19) versehen ist, die ausgebildet ist, um den Stift (21) aufzunehmen, **dadurch gekennzeichnet, dass** die Mittellinie der Bohrung (19) der Blockierbuchse (13) bezüglich der Mittellinie des Gewindes (6) winklig und/oder seitlich versetzt ist.

2. Vorrichtung nach Anspruch 1, **dadurch gekennzeichnet, dass** sie ein Zwischenstück (7) aufweist, das geeignet ist, am feststehenden Element (1) befestigt zu werden, und geeignet ist, zwischen Letzterem und der Blockierbuchse (13) angeordnet zu werden.

3. Vorrichtung nach Anspruch 2, **dadurch gekennzeichnet, dass** das Zwischenstück aus einer Buchse (7) aufgebaut ist, die mit einem Außengewinde (8a) versehen ist, mittels dem sie in das feststehende Element (1) eingeschraubt wird, wobei die Mittellinie (zz') des Außengewindes (8a) dieser Zwischenbuchse mit der Mittellinie des Gewindes (6) zusammenfällt.

4. Vorrichtung nach einem der Ansprüche 1 bis 3, **dadurch gekennzeichnet, dass** sie das feststehende Element aufweist, das aus einer Platte (1) aufgebaut ist, die gegen das Knochenstück (3) aufgesetzt werden kann und dazu bestimmt ist, mit diesem fest verbunden zu werden.

5. Vorrichtung nach einem der Ansprüche 3 oder 4, **dadurch gekennzeichnet, dass** sich der mit Gewinde versehene Teil (13a) der Blockierbuchse (13) nach außen hin fortsetzt mittels eines Greifelementes zum In-Drehung-Versetzen von diesem, und eine Zone geringerer Festigkeit (17) mit gesteuertem Scherbruchpegel zwischen dem mit Gewinde versehenen Teil (13a) und dem Greifelement (13b) vorgesehen ist.

6. Vorrichtung nach Anspruch 4, **dadurch gekennzeichnet, dass** die Zwischenbuchse (7) in ihrem oberen Teil eine kreisförmige Wulst (9) aufweist, die einen Anschlag bildet, der dazu bestimmt ist, auf der Platte (1) zur Anlage zu kommen, und zwar insbesondere in einer Einsenkung (10) von dieser.

7. Vorrichtung nach Anspruch 6, **dadurch gekennzeichnet, dass** die kreisförmige Wulst (9) eine Reihe von Öffnungen (11) aufweist, die dazu dienen, für deren Greifen und deren In-Rotation-Versetzen zu sorgen, um die Zwischenbuchse (7) auf der Platte (1) zu blockieren/zu entblockieren.

8. Vorrichtung nach Anspruch 2, **dadurch gekennzeichnet, dass** sie außerdem ein feststehendes Element aufweist, das aus einem Träger (1a) aufgebaut ist, der die Zwischenstücke (30) aufnimmt, die beweglich montiert sind, und zwar beweglich zum einen entlang dem Profil von diesem und zum anderen bezüglich Rotation, wobei Befestigungseinrichtungen (36) ermöglichen, für eine Immobilisierung der Zwischenstücke (30) bezüglich des feststehenden Elements (1a) zu sorgen.

9. Vorrichtung nach Anspruch 8, **dadurch gekennzeichnet, dass**:
- das feststehende Element aus einer Schiene (1a) aufgebaut ist, deren Innenprofil halbkreisförmig ist und deren Basis von einem in Längsrichtung verlaufenden Langloch (32) durchbrochen ist, das geeignet ist, von den mit Gewinde versehenen Stiften (21) durchquert zu werden,
- die Zwischenstücke aus Kugeln (34) bestehen, die geeignet sind, an einem beliebigen Punkt der Schiene (1a) positioniert zu werden,
- sie ein oberes Kappenelement (36) mit halbkreisförmigem Innenprofil aufweist, dessen Scheitel von einem in Längsrichtung verlaufenden Schlitz (38) durchbrochen ist, der geeignet ist, von mit Gewinde versehenen Stiften (21) durchquert zu werden,
- sie Festspanneinrichtungen (40a, 40b, 42) aufweist, die geeignet sind, die kugelförmigen Zwischenstücke (34) gegen die Schiene (1a) aufzusetzen, um jene bezüglich dieser sowohl in Bezug auf Translation als auch auf Rotation zu immobilisieren.
